# EUROPÄISCHE PATENTSCHRIFT

(11) **EP 3 315 484 B1**
(45) Veröffentlichungstag und Bekanntmachung des Hinweises auf die Patenterteilung: **27.02.2019**
(21) Anmeldenummer: 16198007.3
(22) Anmeldetag: 09.11.2016
(51) Int. Cl.: C07C 29/38, C07C 31/20, C07C 31/22

(54) **VERFAHREN ZUR KOPPELPRODUKTION VON POLYOLEN IN GEGENWART EINER ANORGANISCHEN BASE**
METHOD FOR CO-PRODUCTION OF POLYOLS IN THE PRESENCE OF AN INORGANIC BASE
PROCÉDÉ DE PRODUCTION COUPLÉE DE POLYOLS EN PRÉSENCE D'UNE BASE ANORGANIQUE

(30) Priorität: 25.10.2016 US 201662412306 P
(43) Veröffentlichungstag der Anmeldung: 02.05.2018
(73) Patentinhaber: OXEA GmbH, 46147 Oberhausen (DE)
(72) Erfinder: Frey, Guido D., 64560 Riedstadt (DE); Bothe, Melanie, 40476 Düsseldorf (DE); Strutz, Heinz, 47445 Moers (DE); Slinkard, William E., Richmond, TX 77406 (US)
(74) Vertreter: Michalski Hüttermann & Partner Patentanwälte mbB

(56) Entgegenhaltungen:
- WO-A1-2015/020794

## Beschreibung

Die vorliegende Erfindung betrifft ein Verfahren zur simultan konsekutiven Herstellung von Polyolen durch basenkatalysierte Umsetzung mindestens zweier unterschiedlicher, mittelkettiger Aldehyde mit Formaldehyd. Durch die simultan konsekutive Fahrweise lassen sich hohe Umsätze und hohe Selektivitäten für beide Aldehyde realisieren, wobei zusätzlich eine deutliche Reduzierung der verbleibenden, nicht reagierten Formaldehydmenge erreicht wird. Dies führt zu einer verbesserten Prozessökonomie, da die Energiekosten zur Aufarbeitung des Formaldehydstromes deutlich reduziert werden.

Mehrwertige Alkohole oder Polyole, wie Trimethylolpropan oder Neopentylglykol, besitzen als Kondensationskomponenten zum Aufbau von Polyestern oder Polyurethanen, Kunstharzlacken, Schmiermitteln und Weichmachern eine erhebliche wirtschaftliche Bedeutung. Aus diesem Grund wurden vielfältige industrielle Verfahren entwickelt, welche zu qualitativ hochwertigen und möglichst kostengünstigen Produkten führen sollen. Der Schwerpunkt der Entwicklungen liegt dabei im Wesentlichen auf dem Gebiet derjenigen Prozessparameter, welche einen direkten Einfluss auf die Ausbeute an gewünschtem Hauptprodukt ausmachen. Eine Gesamtbetrachtung der Polyol-Ausbeute unter gleichzeitiger Berücksichtigung der Effizienz des gesamten Edukt- und Energieeinsatzes findet sich hingegen nicht.

Ein möglicher Herstellungsweg zum Erhalt von Polyolen beinhaltet die Umsetzung von Aldehyden mit Formaldehyd in wässriger Lösung. Nach dem Mechanismus einer Aldoladdition kann der Aldehyd in einem ersten Schritt mit Formaldehyd zunächst ein Methylolderivat des entsprechenden Aldehyds bilden. Diese Additionsreaktion kann beispielsweise in Gegenwart katalytischer Mengen an Basen oder Säuren erfolgen. Anschließend kann in einem zweiten Schritt die Aldehydgruppe mit überschüssigem Formaldehyd und mit stöchiometrischen Mengen einer Base in einer Cannizzaro-Reaktion zu der Alkoholgruppe reduziert werden. Als Nebenprodukt entsteht dann gleichzeitig eine stöchiometrische Menge an dem Formiat der zugesetzten Base. Die entsprechenden Formiatsalze fallen als Nebenprodukte an und können beispielsweise als Enteisungsmittel, Bohrhilfsmittel oder als Hilfsmittel in der Lederindustrie verwendet werden.

Für diesen prinzipiellen Umsetzungstyp finden sich in der Patentliteratur vielfältige unterschiedliche Lösungsansätze zur Herstellung von Polyolen.

So wird zum Beispiel die Herstellung von Trimethylolpropan nach einem anorganischen Cannizzaro-Prozess in der DE 1 182 646 A, WO 99/20586 A1, EP 2 341 041 A1, EP 1 323 698 A2, WO 2015/020796 A1 oder WO 2015/020794 A1 offenbart. Die zugrundeliegende Reaktion zwischen n-Butyraldehyd und Formaldehyd ist stark exotherm und die freigesetzte Wärme führt zu nachteiligen Temperaturspitzen, die die Selektivität der Reaktion beeinflussen und zu Farbproblemen im Endprodukt führen können. Die Reaktion wird üblicherweise in Gegenwart einer großen Wassermenge mit entsprechend hoher Wärmekapazität geführt, um die Reaktionswärme aufzunehmen. Die große Wassermenge wird durch den Einsatz einer vergleichsweise verdünnten wässrigen Formaldehydlösung, sowie durch den Einsatz wässriger Lösung anorganischer Basen erreicht.

Nach der DE 1 182 646 A1 ist es ebenfalls vorteilhaft, einen hohen Überschuss an Formaldehyd zu verwenden, um die Ausbeute an Trimethylolpropan zu erhöhen und die Bildung unerwünschter Nebenprodukte, die zu Farbbeeinträchtigungen führen können, zurückzudrängen. Bezogen auf den n-Butyraldehydeinsatz wird angegeben, dass man mit 6 bis 10 Molen Formaldehyd arbeiten kann. Ferner wird empfohlen, die anorganische Base in einer Menge über die theoretisch erforderliche Menge hinaus einzusetzen.

Eine Herstellungsweise mit sukzessiver Zugabe der Edukte wird beispielsweise in der WO 2015/020796 A1 offenbart. Zur Herstellung von Trimethylolpropan wird eine Reaktionsführung in einem Rohrreaktor vorgeschlagen, in welcher weitere Reaktionspartner entlang des Rohrreaktors im Verlaufe des Reaktionsfortschritts stufenweise zugegeben werden. Dabei wird dem Rohrreaktor ein formaldehydhaltiger Strom zugeführt und über die Länge des Rohrreaktors n-Butyraldehyd und eine wässrige Lösung einer anorganischen Base an verschiedenen Stellen des Rohres hinzugegeben. Durch die stufenweise Zugabe von n-Butyraldehyd zu dem formaldehydhaltigen Strom wird stets ein hoher Überschuss an Formaldehyd, bezogen auf n-Butyraldehyd, gewährleistet und die Selektivität in Richtung Trimethylolpropan begünstigt. An den Zugabestellen von n-Butyraldehyd und anorganischer Base kann der Rohrreaktor statische Mischer oder Einbauten enthalten, um nach Eintritt der Reaktionspartner das Vermischen zu intensivieren und die Reaktionswärme abzuführen.

Die bekannten Verfahren zur Herstellung von Polyolen nach dem Cannizzaro-Verfahren in Gegenwart anorganischer Basen arbeiten mit einem erheblichen Überschuss an Formaldehyd, der meistens in Form einer verdünnten wässrigen Lösung der Reaktion zugeführt wird. Nach der Reaktion ist aus dem Produkt-Rohgemisch daher eine erhebliche Menge an nicht reagiertem Formaldehyd abzutrennen. Infolge des hohen Wasseranteils wird Formaldehyd als stark verdünnte, wässrige Lösung abgetrennt, wozu ein entsprechend hoher Energieaufwand erforderlich ist.

Es ist die Aufgabe der vorliegenden Erfindung, ein verbessertes Herstellungsverfahren bereitzustellen, welches es erlaubt, unter hohen Raum-Zeitausbeuten an Polyolen den Energieeinsatz zur Abtrennung wässriger Formaldehydlösung aus dem erhaltenen rohen Reaktionsprodukt zu reduzieren.

Erfindungsgemäß gelöst wird die Aufgabe durch ein Verfahren zur simultan konsekutiven Herstellung mindestens zweier unterschiedlicher Polyole durch Umsetzung aliphatischer Aldehyde mit Formaldehyd in Gegenwart einer anorganischen Base, wobei in einem 1. Verfahrensschritt ein aliphatischer C2-C9 Aldehyd mit Formaldehyd in Gegenwart einer anorganischen Base zur Reaktion gebracht und ohne Aufarbeitung der Reaktionslösung in einem 2. Verfahrensschritt die Reaktionslösung aus dem 1. Verfahrensschritt unter Zugabe zumindest eines vom Aldehyd des 1. Verfahrensschrittes unterschiedlichen aliphatischen C2-C9 Aldehyds weiter zur Reaktion gebracht wird. Überraschenderweise hat sich gezeigt, dass durch diese zwei-schrittige Verfahrensführung, in welcher die beiden aliphatischen Aldehyde gleichzeitig (simultan) aber nacheinander (konsekutiv) zur Reaktion gebracht werden, sich die gewünschten Polyole mit einem hohen Umsatz und einer hohen Selektivität erhalten lassen. Des Weiteren ist herauszuheben, dass durch die Kopplung der Synthesestufen zusätzlich die Restbelastung des Reaktionswassers am Ende beider Schritte mit Formaldehyd deutlich verringert wird. Gerade letzteres führt zu einer deutlichen Steigerung der Prozessökonomie, da der Energieaufwand zu Wiedergewinnung des Formaldehyds aus der wässrigen Lösung nach der Reaktion deutlich reduziert wird. Diese Verfahrensführung ist zudem flexibel einsetzbar, wobei auch in den unterschiedlichsten Reaktortypen sowohl innerhalb einer kontinuierlichen als auch innerhalb einer Batchherstellung hohe Raum-Zeitausbeuten zugänglich sind. Ferner enthält das aus dem ersten Verfahrensschritt abfließende Reaktionsgemisch bereits eine erhebliche Wassermenge und die Gefahr der Bildung von Temperaturspitzen bei einer exothermen Umsetzung im zweiten Verfahrensschritt wird dadurch deutlich gemindert. Ein eventuell nötiger, zusätzlicher Wassereintrag innerhalb des zweiten Verfahrensschrittes kann daher deutlich reduziert werden. Diese Vorteile waren insgesamt von vorneherein nicht zu erwarten, da es sich in den einzelnen Verfahrensschritten jeweils um komplexe Umsetzungen handelt, wobei ein Einfluss weiterer Synthesekomponenten auf den einzelnen Umsetzungsgrad, die Selektivität und das Nebenproduktprofil im hohen Maße anzunehmen ist. Das gerade die Reaktion der beiden unterschiedlichen Aldehyde eher unabhängig voneinander, unter weitgehenden Umsatz der Reaktionskomponente Formaldehyd abläuft, war durch die unterschiedlichen Reaktivitäten und Löslichkeiten der Aldehyde im Reaktionsmedium und aufgrund möglicher Kreuzreaktionen zwischen beiden Aldehyden oder Folgeprodukten der Aldehyde untereinander oder mit noch vorhandenem Aldehyd überraschend.

Am Beispiel der Darstellung von Trimethylolpropan und Neopentylglykol aus n-Butyraldehyd und iso-Butyraldehyd ergibt sich eine mögliche Ausgestaltung der Reaktionsführung nach:

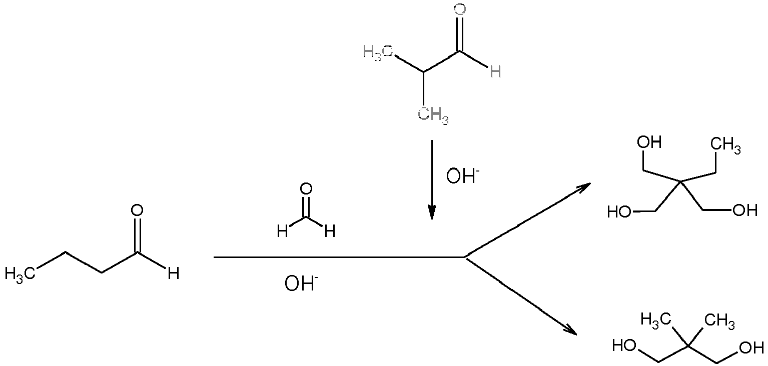

Das erfindungsgemäße Verfahren basiert dabei auf einer simultan konsekutiven Herstellung mindestens zweier unterschiedlicher Polyole. Dies bedeutet insbesondere, dass die Herstellung der Polyole nicht unabhängig voneinander durchgeführt wird, sondern dass ein Eduktaldehyd zuerst innerhalb einer Reaktion mit Formaldehyd umgesetzt und zu einem späteren Zeitpunkt mindestens ein zweites Aldehyd zu der gesamten Reaktionslösung des ersten Aldehyds hinzugegeben wird, also eine im Wesentlichen sequenzielle oder simultan konsekutive Reaktionsführung. Zumindest innerhalb einer Zeitspanne findet dann in der Reaktionslösung sowohl eine Umsetzung des einen wie auch des anderen Aldehyds statt. Die Umsetzung der unterschiedlichen Aldehyde erfolgt also zumindest partiell gleichzeitig und innerhalb derselben Reaktionslösung. Weiterhin liegt der Zeitpunkt der gleichzeitigen Umsetzung insbesondere nicht zu Anfang der Umsetzung des ersten Aldehyds. Umsetzungen, in denen von vorneherein 2 unterschiedliche Aldehyde als Reaktionspartner des Formaldehyds in signifikanten Mengen in einer Reaktionslösung vorliegen oder in denen eine Gesamtmenge zweier unterschiedlicher Aldehyde neben dem Formaldehyd gemeinsam zu einer Lösung hinzugefügt werden, also im Wesentlichen eine simultane Umsetzung der beiden Aldehyde mit Formaldehyd, sind nicht im Sinne der Erfindung. Ebenso wenig ist es im Sinne der Erfindung, wenn ein erster Aldehyd geringe Restmengen eines zweiten Aldehyds aufweist und beide gleichzeitig mit Formaldehyd zur Reaktion gebracht werden. Durch das erfindungsgemäße Verfahren werden jeweils signifikante Mengen zweier unterschiedlicher Polyole erhalten. Die Herstellungsmengen der beiden unterschiedlichen Polyole sind jeweils signifikant, wenn sie zum Beispiel ungefähr gleich sind oder wenn das Molverhältnis zwischen diesen am Ende des 2. Verfahrensschrittes größer oder gleich 20 %, bevorzugt größer oder gleich 50 % und des Weiteren bevorzugt größer oder gleich 80 % beträgt.

Innerhalb des erfindungsgemäßen Verfahrens werden mindestens zwei unterschiedliche Polyole hergestellt. Die herstellbaren Polyole weisen insbesondere mindestens zwei OH-Gruppen auf. Die chemische Zusammensetzung der Polyole ist natürlich eine Funktion der eingesetzten Aldehyde und des Umsetzungsgrades im Rahmen der Aldoladdition und anschließenden Cannizarro-Reaktion. Mögliche durch dieses Verfahren erhältliche Polyole sind beispielsweise Pentaerythritol, 1,1,1-Tris(hydroxymethyl)ethan (TME), 1,1,1-Tris(hydroxymethyl)propan (TMP), 1,1,1-Tris(hydroxymethyl)butan (TMB), 1,1,1-Tris(hydroxymethyl)pentan, 1,1,1-Tris(hydroxymethyl)hexan, 1,1,1-Tris(hydroxymethyl)heptan, 1,1,1-Tris(hydroxymethyl)oktan, 1,1,1-Tris(hydroxymethyl)-2-methylpropan, 1,1,1-Tris(hydroxymethyl)-2,2,4-trimethylpentan, Neopentylglykol, 2-Propylpropan-1,3-diol, 2-Methyl-2-propylpropan-1,3-diol, 2-Ethyl-2-methylpropan-1,3-diol, 2-Butyl-2-ethylpropan-1,3-diol, 2-Butyl-2-methylpropan-1,3-diol, 2-Pentylpropan-1,3-diol, 2-Hexyl-2-methylpropan-1,3-diol, 2-Hydroxymethyl-2-(1,3,3-trimethylbutyl)propan-1,3-diol, 2-(3,3-Dimethylbutyl)-2-methylpropan-1,3-diol.

Im Rahmen der erfindungsgemäßen Umsetzung werden mindestens zwei unterschiedliche aliphatische Aldehyde neben Formaldehyd eingesetzt. Dies bedeutet, dass mindestens zwei chemisch verschiedene Aldehyde mit Formaldehyd zur Reaktion gebracht werden, wobei die Aldehyde sich prinzipiell in ihrer Summenformel und/oder in ihrer sterischen Ausgestaltung unterscheiden können. Es werden aliphatische Aldehyde zur Reaktion gebracht, also Aldehyde, welche neben der Aldehydgruppe noch nicht-aromatische Kohlenwasserstoffreste tragen. Die nicht aromatischen Reste können insbesondere substituierte oder nicht substituierte C1-C8 Kohlenwasserstoffketten sein. Unter substituierten Kohlenwasserstoffketten werden Kohlenwasserstoffketten verstanden, in denen maximal 2 Wasserstoffatome durch Heteroatome wie O, Halogene, N oder die dem Fachmann geläufigen Heteroatomgruppen enthaltend eines dieser Heteroatome, ausgetauscht sind.

Die beiden unterschiedlichen Aldehyde werden mit Formaldehyd umgesetzt. Zweckmäßigerweise kann das zur Reaktion bringen durch Zusatz einer wässrigen Formaldehydlösung erfolgen. Der Formaldehydgehalt in der wässrigen Lösung kann 5 bis 50 Gew.-%, vorzugsweise 8 bis 35 Gew.-% und insbesondere 9 bis 30 Gew.-% betragen.

Die Reaktion des Aldehyds mit dem Formaldehyd findet in Gegenwart einer anorganischen Base statt. Als anorganische Base eignen sich insbesondere basische Alkali- oder Erdalkalimetallverbindungen, welche als wässrige Lösung oder als wässrige Suspension der Reaktionslösung zugesetzt werden können.

In dem 1. Verfahrensschritt wird ein aliphatischer C2-C9 Aldehyd mit Formaldehyd in Gegenwart einer anorganischen Base zur Reaktion gebracht. In dieser Reaktion einsetzbare C2-C9 Aldehyde sind beispielsweise aliphatische n-Aldehyde oder verzweigte aliphatische Aldehyde. Bevorzugt können aber insbesondere Acetaldehyd, Propanal, n-Butanal, iso-Butanal, Valeraldehyd, 2-Methylbutanal, 3-Methylbutanal, n-Hexanal, 2-Methylpentanal, n-Heptanal, 2-Methylhexanal, n-Octanal, 2-Ethylhexanal, n-Nonanal, 2-Methyloktanal, 3,5,5-Trimethylhexanal, 2,5,5-Trimethylhexanal eingesetzt werden. Diese Aldehyde bilden die organische Phase, wobei - ohne durch die Theorie gebunden zu sein - durch den Kontakt mit oder durch Eindiffundieren in die wässrige Phase es zu einer Aldoladdition mit dem dort vorliegenden Formaldehyd unter Katalyse durch die gelöste oder dispergierte Base kommt. Das zur Reaktion bringen kann dabei beinhalten, dass mindestens 10, 30, 50, 75 oder 100 mol-% des aliphatischen Aldehyds im 1. Verfahrensschritt umgesetzt werden. Es ist also möglich, die Reaktion auf einen kompletten oder Teilumsatz des Aldehyds des 1. Verfahrensschrittes zu fahren. Bevorzugt liegen aber am Ende des 1. Verfahrensschrittes noch 5, 15, 20, 40, 60 mol-% des eingesetzten Aldehyds in der Lösung vor. Der im 1. Verfahrensschritt zugesetzte Aldehyd kann sowohl in einer einmaligen Gabe, aufgeteilt in mehrere Stufen oder kontinuierlich der Reaktionslösung zugegeben werden.

Die im 1. Verfahrensschritt erhaltene Reaktionslösung wird anschließend ohne Aufarbeitung der Reaktionslösung direkt dem 2. Verfahrensschritt zugeführt. Bei dieser gekoppelten Verfahrensführung dient somit das aus dem 1. Verfahrensschritt abfließende Reaktionsgemisch als Einsatzstrom für den 2. Verfahrensschritt. Insbesondere bedeutet dies, dass die Reaktionslösung aus dem 1. Verfahrensschritt ohne weitere thermische, chemische oder mechanische Trenn- oder Aufbereitungsoperationen, d.h. insbesondere ohne Destillation, Absetzen lassen o.ä. dem 2. Verfahrensschritt zugeführt wird. Es werden also zwischen den beiden Verfahrensschritten insbesondere keine weiteren Substanzen der Reaktionslösung entnommen. Falls die beiden Verfahrensschritte in unterschiedlichen Reaktoren durchgeführt werden, so ist ein Überführen, beispielsweise durch Umpumpen der Reaktionslösung, aus dem einen in einen anderen Reaktor jedoch möglich.

Im 2. Verfahrensschritt wird die Reaktionslösung aus dem 1. Verfahrensschritt zumindest unter Zugabe eines vom Aldehyd des 1. Verfahrensschrittes unterschiedlichen C2-C9 Aldehyds weiter zur Reaktion gebracht. Bevorzugt können im 2. Reaktionsschritt in Position-2 verzweigte Aldehyde, wie z.B. i-Butanal, verwendet werden. Der 2. Verfahrensschritt startet also definitionsgemäß zu dem Zeitpunkt, in dem der Reaktionslösung des 1. Verfahrensschrittes ein weiterer, anderer C2-C9 Aldehyd zugegeben wird. Prinzipiell lassen sich als Aldehyde des 2. Verfahrensschrittes dieselben Aldehyde einsetzen wie weiter oben beschrieben, nur mit der Maßgabe, dass die eingesetzten Aldehyde des 1. und des 2.

Verfahrensschrittes unterschiedlich sind. Neben den Aldehyden können im 2. Verfahrensschritt auch weitere Substanzen wie beispielsweise weitere Base oder auch Formaldehyd zugegeben werden. Innerhalb des 2. Verfahrensschrittes reagiert dann der neu hinzugegebene Aldehyd und der noch nicht umgesetzte Aldehyd des 1. Verfahrensschrittes mit dem verbliebenen (oder ggf. neu zugesetzten) Formaldehyd unter Bildung mindestens eines weiteren, unterschiedlichen Polyols. Bevorzugt wird die Reaktion derart gesteuert, dass für beide Aldehyde Vollumsatz erreicht wird.

In einer ersten bevorzugten Ausführungsform kann im 1. Verfahrensschritt ein an 2-Position nicht-verzweigter aliphatischer C2-C9 Aldehyd und im 2. Verfahrensschritt ein in 2-Position verzweigter aliphatischer C2-C9 Aldehyd zur Reaktion gebracht werden. Es hat sich als besonders günstig erwiesen, wenn die Verfahrensführung so gewählt wird, dass im 1. Verfahrensschritt ein in 2-Position nicht verzweigter Aldehyd und im 2. Verfahrensschritt die in 2-Position verzweigte iso-Form desselben oder eines anderen Aldehyds eingesetzt wird. Durch diese Zugabe lassen sich besonders hohe Selektivitäten und Umsätze erhalten. Zudem kann man zweckmäßigerweise die Reaktion derart steuern, dass zwei unterschiedlich funktionale Alkohole als Hauptprodukte erhältlich sind. In besonders hohe Ausbeuten und ohne die Bildung nennenswerter Nebenprodukte lassen sich mindestens 2 unterschiedliche Polyole erhalten, wobei die Hauptmenge der gebildeten Alkohole aus Di- und Triolen besteht. In einer weiterhin bevorzugten Verfahrensvariante kann der im 1. Verfahrensschritt zugesetzte Aldehyd zu einem dreiwertigen und der in dem 2. Verfahrensschritt zugesetzte Aldehyd zu einem zweiwertigen Alkohol umgesetzt werden.

In einer zweiten bevorzugten Ausgestaltung kann der im 1. Verfahrensschritt eingesetzte Aldehyd aus der Gruppe bestehend aus n-Propanal, n-Butyraldehyd, Valeraldehyd ausgesucht und der im 2. Verfahrensschritt eingesetzte Aldehyd iso-Butyraldehyd sein. Insbesondere die gekoppelte Umsetzung eines gradkettigen n-Aldehyds im 1. mit einem in α-Stellung zur Aldehydgruppe verzweigten Aldehyd im 2. Verfahrensschritt kann zu einer besonders effektiven Verfahrensführung mit einer geringeren Anzahl unerwünschter Koppelprodukte beitragen.

Innerhalb eines weiteren Aspektes des erfindungsgemäßen Verfahrens können der 1. und der 2. Verfahrensschritt bei einer Temperatur von größer oder gleich 10 °C und kleiner oder gleich 105 °C durchgeführt werden. Trotz des Einsatzes chemisch unterschiedlicher Aldehyde und dem komplexen Zusammenspiel der Edukte innerhalb einer gemeinsamen Reaktionslösung hat sich überraschenderweise herausgestellt, dass die gekoppelte Reaktion innerhalb eines gemeinsamen, oben angegebenen Temperaturbereiches, durchgeführt werden kann. Innerhalb dieses Temperaturbereiches lassen sich ausreichende Umsätze bei hinreichenden Selektivitäten für beide Aldehyde erreichen, so dass die Gesamtumsetzung an Aldehyden mit einer guten Raum-Zeitausbeute erreicht wird. Insbesondere der 1. Verfahrensschritt kann dabei mit einer Temperatur von größer oder gleich 20 °C und kleiner oder gleich 65 °C und der 2. Verfahrensschritt bei einer Temperatur von größer oder gleich 30 °C und kleiner oder gleich 75 °C durchgeführt werden. Innerhalb dieses engeren Bereiches lassen sich besonders hohe Raum-Zeitausbeuten selbst für die unterschiedlichsten Reaktortypen realisieren.

In einem weiteren, vorteilhaften Aspekt des erfindungsgemäßen Verfahrens kann im 2. Verfahrensschritt zusätzlich eine anorganische Base zur Reaktionslösung zugegeben werden. Zum Erhalt hoher Umsätze und Selektivitäten hat es sich als besonders vorteilhaft erwiesen, dass nicht die gesamte Basenmenge für die gekoppelte Reaktion innerhalb des 1. Verfahrensschrittes zugegeben, sondern, dass ein Teil der zur Abreaktion benötigten Base erst im 2. Verfahrensschritt zugesetzt wird. Dies kann die Selektivität des 1. Verfahrensschrittes erhöhen, da die Gesamtmenge an Base im 1. Verfahrensschritt kleiner gehalten werden kann. Diese Verfahrensführung ermöglicht zudem den Einsatz insgesamt geringerer Wassermengen, da die Base im 2. Verfahrensschritt in einer konzentrierteren Lösung zugegeben werden kann. Somit kann die zugegebene Gesamtwassermenge reduziert werden, welches zu einer verbesserten Prozessökonomie beitragen kann.

In einer weiteren Ausführungsform kann im 1. und 2. Verfahrensschritt dieselbe anorganische Base eingesetzt werden. Trotz der unterschiedlichen Reaktivitäten der unterschiedlichen Aldehyde und der unterschiedlichen Prozessbedingungen bei der Umsetzung im 1. und 2. Verfahrensschritt hat es sich als möglich und geeignet erwiesen, die Umsetzungen mit derselben anorganischen Base durchzuführen. Es lassen sich durch diese Prozessführung gute Selektivitäten und gute Umsätze erhalten und zusätzlich erleichtert der Einsatz nur einer Base die anschließenden Trennoperationen zur Aufreinigung der Haupt- und Nebenprodukte.

In einer weiteren Ausgestaltung des Verfahrens kann die anorganische Base aus der Gruppe bestehend aus NaOH, KOH, Ca(OH)₂ oder Mischungen daraus ausgesucht sein. Die angeführte Gruppe an anorganischen Basen führt im Rahmen der erfindungsgemäßen Prozessführung zu hohen Produktumsätzen und -selektivitäten. Zudem lässt sich aufgrund der Löslichkeit dieser Basen auch der Wassergehalt der Reaktionslösung optimieren, sodass in Summe nach der Reaktion eine geringere Wassermenge anfällt. Vorzugsweise kann in der ersten und zweiten Reaktionsstufe die gleiche anorganische Base verwendet werden. Da im zweiten Verfahrensschritt bereits eine hohe Wassermenge vorliegt, kann die anorganische Base in vergleichsweise hoher Konzentration eingesetzt werden. Bewährt haben sich wässrige Lösungen oder Suspensionen mit einer Konzentration von 15 bis 52 Gew.-%, vorzugsweise von 30 bis 50 Gew.-% an anorganischer Base. Zweckmäßigerweise verwendet man kommerziell erhältliche wässrige Lösungen an anorganischer Base mit entsprechend hoher Konzentration. Besonders geeignet sind wässrige Kaliumhydroxid- oder Natriumhydroxidlösungen oder eine wässrige Suspension von Calciumhydroxid.

In einem zusätzlichen Verfahrensaspekt kann das Molverhältnis anorganischer Base zu aliphatischen Aldehyd in den beiden Verfahrensschritten größer oder gleich 1 zu 1 und kleiner oder gleich 1,6 zu 1 betragen. Trotz der aktiven Veränderung der Reaktionslösung durch weitere Zugabe eines anderen Aldehyds innerhalb des 2. Verfahrensschrittes wurde gefunden, dass vorteilhafterweise die Basenkonzentration innerhalb beider Verfahrensschritte annähernd gleich gewählt werden kann. Diese Basenkonzentration führt zu einer hinreichenden Effizienz beider Teilreaktionen mit hohen Raum-Zeitausbeuten und ermöglicht eine einfache Prozessführung unter nur geringen Energiekosten zur Aufarbeitung der Reaktionslösung.

Innerhalb einer weiteren Verfahrensvariante kann der Formaldehyd im 1. Verfahrensschritt in Form einer wässrigen Formaldehydlösung mit einem Formaldehydgehalt von größer oder gleich 5 Gew.-% und kleiner oder gleich 50 Gew.-% zugegeben werden. Die Zugabe des Formaldehyds in Form einer wässrigen Lösung ermöglicht neben der Bereitstellung eines zweiphasigen Reaktionssystems auch eine effektive Temperaturkontrolle der Umsetzung im 1. Verfahrensschritt. Durch die Wärmekapazität des Wassers bedingt, kann in diesem Konzentrationsbereich an Formaldehyd ein ausreichender Umsetzungsgrad unter gleichzeitiger Sicherstellung einer hinreichenden Reaktionsgeschwindigkeit und nur geringer Nebenproduktbildung erreicht werden. Vorteilhafterweise kann im 1. Verfahrensschritt eine 10 bis 40 Gew.-% oder weiterhin bevorzugt eine 15 bis 25 Gew.-%ige Formalinlösung verwendet werden.

Nach einer weiteren, erfindungsgemäßen Ausgestaltung kann das Molverhältnis Formaldehyd zu aliphatischem Aldehyd im 1. Verfahrensschritt größer oder gleich 3,1 zu 1 und kleiner oder gleich 12 zu 1 und das Molverhältnis Formaldehyd zu aliphatischem Aldehyd im 2. Verfahrensschritt größer oder gleich 2,1 zu 1 und kleiner oder gleich 7 zu 1 betragen. Ein hoher molarer Überschuss an Formaldehyd, bezogen auf den im 1. Verfahrensschritt zugesetzten Aldehyd kann die Selektivität der Umsetzung des Aldehyds zu einem Polyol deutlich begünstigen. Ist es gewünscht insbesondere aus dem im 1. Verfahrensschritt zugesetzten Aldehyd einen dreiwertigen Alkohol herzustellen, so kann in der ersten Reaktionsstufe über die stöchiometrisch erforderliche Menge von 3 Molen Formaldehyd mit 4 bis 12, vorzugsweise mit 5 bis 10 Molen Formaldehyd, bezogen auf 1 Mol Aldehyd gearbeitet werden. In einer bevorzugten Ausgestaltung kann man eine wässrige Formaldehydlösung in einer Konzentration von 9 bis 30 Gew.-% und in einer Menge von 5 bis 10 Molen, bezogen auf ein Mol im 1. Verfahrensschritt eingesetzten Aldehyds verwenden.

Nach einer weiteren Ausgestaltung des Verfahrens kann der Formaldehyd nur im 1. Verfahrensschritt zugegeben werden. Zur Kontrolle der Temperatursteuerung der exothermen Umsetzung im 1. Verfahrensschritt und zum Erhalt einer möglichst selektiven Umsetzung kann es sinnvoll sein, die gesamte für den 1. und 2. Verfahrensschritt benötigte Menge an Formaldehyd schon am Anfang des 1. Verfahrensschrittes zuzugeben. In dieser Ausgestaltung erhält man eine schnelle Umsetzung mit hohen Umsätzen des ersten Aldehyds, wobei insbesondere mehrwertige Alkohole mit 3 OH-Gruppen sehr effizient gebildet werden können. Durch die mit dem Formaldehyd eingetragene Wassermenge lassen sich zudem Nebenreaktionen effektiv verringern, welche in Anbetracht von Temperaturspitzen auftreten können. Weiterhin wird in dieser Verfahrensvariante durch nur den Zusatz des weiteren Aldehyds im 2. Verfahrensschritt eine sehr effektive Reduktion des Restformaldehyds aus dem 1. Verfahrensschritt erreicht. Die Reaktion des im 1. Verfahrensschritt zugegebenen Aldehyds kann also durch den hohen molaren Formaldehydüberschuss gezielt gesteuert werden. Zweckmäßigerweise kann der im 2.

Verfahrensschritt zugegebene Aldehyd unter Verbrauch des Restformaldehydgehaltes (oder eines Teiles davon) zu einem Polyol mit geringerer OH-Gruppenzahl hocheffizient abreagieren. Bei geeigneter Wahl der Gesamtmenge an Formaldehyd ist dies mit einer Minimierung des Formaldehyd-Recycles inklusive einer Minimierung des zu entfernenden Wassers in der Aufarbeitung verbunden.

Innerhalb eines weiteren Aspektes des erfindungsgemäßen Verfahrens kann die Zugabe mindestens eines Eduktes im 1. und/oder im 2. Verfahrensschritt stufenweise erfolgen. Zur Steuerung der Selektivität der Reaktionen der einzelnen Aldehyde kann es sich als günstig erweisen, wenn mindestens eines der Edukte stufenweise der Reaktionslösung zugegeben wird. Eine stufenweise Zugabe erfolgt insbesondere dann, wenn die Zusammensetzung der Reaktionslösung im 1. Verfahrensschritt nicht konstant ist, sondern die Konzentration eines der Edukte im 1. Verfahrensschritt als Funktion der Zeit relativ zu den anderen Edukten erhöht wird. Beispielsweise kann dies dadurch erfolgen, indem eines der Edukte in mehreren Stufen oder Portionen der Reaktionslösung zugegeben wird. Die Zugabe kann am selben Ort aber zu unterschiedlichen Zeiten oder an unterschiedlichen Orten zu unterschiedlichen Zeiten erfolgen. Auf diese Art und Weise lassen sich die Reaktionsgleichgewichte beeinflussen und die Selektivität und der Umsetzungsgrad der Reaktion steuern.

Nach einer weiteren, bevorzugten Ausführungsform kann der aliphatische Aldehyd im 1. Verfahrensschritt stufenweise der Reaktionsmischung zugegeben werden. Gerade der stufenweise Einsatz des Aldehyds innerhalb des 1. Verfahrensschrittes kann zu einer höheren Selektivität und Verringerung der Bildung unerwünschter Nebenprodukte beitragen. Dies kann eventuell an einer verbesserten Temperaturkonstanz liegen, da durch den stufenweisen Eintrag die Umsetzung kontrollierter ist und Temperaturspitzen vermieden werden.

Nach einer bevorzugten Ausgestaltung des erfindungsgemäßen Verfahrens kann der Umsatz an Aldehyd in der Reaktionsmischung am Ende des 1. Verfahrensschrittes größer oder gleich 50% betragen. Die Zugabe des 2. Aldehyds im 2. Verfahrensschritt erfolgt also in dieser Ausgestaltung erst dann, wenn der Umsatz des 1. Aldehyds aus dem 1. Verfahrensschritt schon mindestens 50% beträgt. Auf diese Weise ist schon ein substantieller Teil des Formaldehyds abreagiert und der 2. Aldehyd und der Rest des im 1. Verfahrensschritt zugegebenen 1. Aldehyds reagieren in einer Reaktionslösung, welche deutlich geringere Formaldehydkonzentrationen aufweist. Dies kann die Selektivität der Reaktionen beeinflussen und kann insbesondere dazu führen, dass man den Restgehalt des Formaldehyds in der Reaktionslösung am Ende des 2. Verfahrensschrittes sehr genau steuern kann. Diese Verfahrensvariante kann zu besonders geringen Aufarbeitungskosten der fertigen Reaktionslösung beitragen. Des Weiteren kann durch einen schon hohen Umsetzungsgrad im 1. Verfahrensschritt die zusätzlich ins System einzutragende Wasser- und Wärmemenge reduziert werden.

In einer weiteren Ausführungsform kann das zur Reaktion bringen im 1. und 2. Verfahrensschritt in einem oder mehreren getrennten Reaktoren erfolgen, wobei die Reaktoren ausgesucht sind aus der Gruppe bestehend aus Rohr-, Rohrbündel-, Plattenreaktoren, Rührkesseln oder einer Kombination daraus. Diese Reaktortypen ermöglichen durch ihre spezifische Wärmeaustauschfläche eine besonders gute thermische Reaktionssteuerung, wobei insbesondere hohe Kühlleistungen pro Reaktionsabschnitt möglich sind. Zusätzlich ermöglichen diese Reaktoren eine gute Durchmischung unter Erhalt hoher Reynolds-Zahlen und ein sehr homogenes Verweilzeitverhalten der Reaktionsmischung.

Innerhalb eines zusätzlichen, erfindungsgemäßen Aspektes kann das durchgesetzte Eduktvolumen pro Reaktorvolumen und Zeit (V/Vh) in beiden Verfahrensschritten größer oder gleich 0,3 und kleiner oder gleich 4,0 betragen. Diese Fahrweise kann zu einem ausreichenden Produktumsatz und zu einer Reduktion der Anlagenkosten beitragen.

In einer weiteren Ausgestaltung können in den beiden Verfahrensschritten Aldehyde mit derselben Elementzusammensetzung eingesetzt werden, wobei der im 1. Verfahrensschritt zugesetzte Aldehyd ein Strukturisomer des im 2. Verfahrensschritt zugesetzten Aldehyds ist.

In einer weiteren Ausführungsform können in den beiden Verfahrensschritten Aldehyde mit derselben Elementzusammensetzung eingesetzt werden, wobei der im 1. Verfahrensschritt zugesetzte aliphatische Aldehyd ein Strukturisomer des im 2. Verfahrensschritt zugesetzten Aldehyds ist und der im 1. Verfahrensschritt zugesetzte Aldehyd ein in alpha-Position unverzweigter Aldehyd und der im 2. Verfahrensschritt zugesetzte Aldehyd ein in 2-Position verzweigter Aldehyd ist. Bevorzugt können n-Butanal und iso-Butanal eingesetzt werden.

In einer weiteren Ausgestaltung des Verfahrens kann die Reaktion unter Strömungsbedingungen betrieben werden, welche entweder im Übergangsbereich zwischen laminarer und turbulenter Strömung oder im turbulenten Regime liegen. Dieser Strömungsbereich hat sich als sehr geeignet erwiesen, eine effektive Temperaturkontrolle beider Reaktionen in den beiden Verfahrensschritten zu gewährleisten. Dies kann sich positiv auf das Nebenproduktprofil und -die Nebenproduktmenge auswirken. Zudem stellt dieser Strömungsbereich anscheinend eine geeignete Phasengrenzfläche bereit, welche Kreuzreaktionen zwischen den beiden Aldehyden weitgehend vermeidet.

Weitere Einzelheiten, Merkmale und Vorteile des Gegenstandes der Erfindung ergeben sich aus den Unteransprüchen sowie aus der nachfolgenden Beschreibung der Figur und der zugehörigen Beispiele. Es zeigt die:

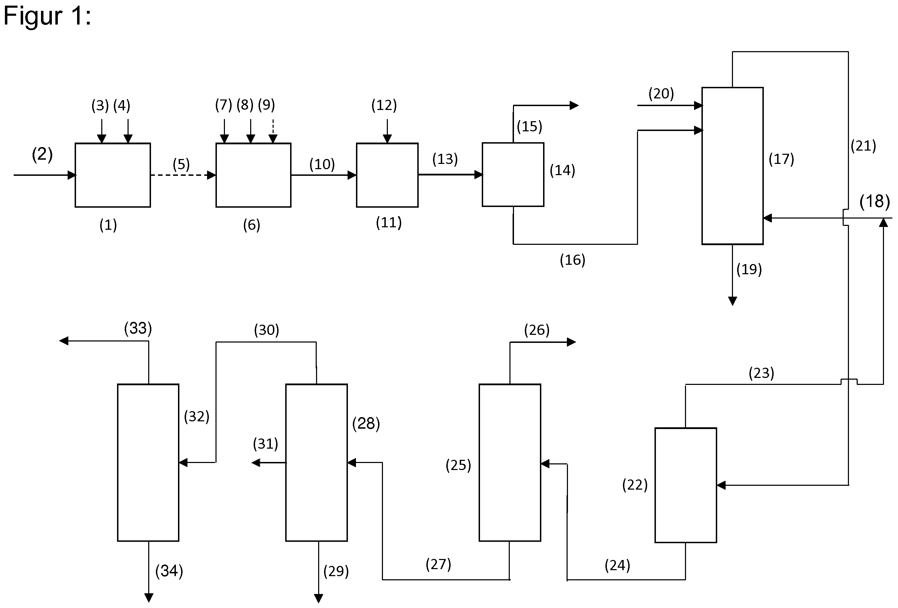

Fig. 1 schematisch eine mögliche apparative Ausgestaltung des erfindungsgemäßen Verfahrens am Beispiel einer gemeinsamen Herstellung von Trimethylolpropan und Neopentylglykol

### Apparativer Aufbau zur Synthese der Verbindungen

Die Figur 1 zeigt ein mögliches Aufbauschema für die Koppelproduktion von Trimethylolpropan und Neopentylglykol in zwei Reaktionszonen. Die beiden Reaktionszonen werden gekoppelt voneinander betrieben, wobei das Reaktionsgemisch aus der ersten Reaktionszone als Einsatzstrom für die zweite Reaktionszone dient. Zusätzlich ist auch ein weiterer möglicher Weg für die nachfolgende Aufarbeitung des Trimethylolpropan- und Neopentylglykolhaltigen Reaktionsgemisches aufgezeigt.

Der ersten Reaktionszone (1) wird über die Leitung (2) ein wässriger formaldehydhaltiger Einsatzstrom zugeführt, über die Leitung (3) n-Butyraldehyd und über die Leitung (4) eine wässrige Lösung einer anorganischen Base wie Natriumhydroxid oder Kaliumhydroxid. Die Umsetzung kann bei Temperaturen von 30 bis 60°C, vorzugsweise von 40 bis 55°C erfolgen. Anschließend kann das Reaktionsgemisch ohne weitere Aufarbeitung in eine zweite Reaktionszone (6) gegeben werden. Die erste und zweite Reaktionszone können optional über die Leitung (5), gestrichelt dargestellt, verbunden sein. Es ist aber auch möglich, die erste und zweite Reaktionszone in einem Mehrzonenreaktor zu vereinen. Neben dem aus der ersten Reaktionszone herangeführten Reaktionsgemisch kann der zweiten Reaktionszone über Leitung (7) iso-Butyraldehyd und über Leitung (8) die wässrige Lösung der anorganischen Base zugeführt werden. Optional kann über die Leitung (9), gestrichelt dargestellt, eine wässrige Formaldehydlösung in die zweite Reaktionszone (6) gegeben werden. Die zweite Reaktionszone kann bei einer Temperatur von 40 bis 70°C, vorzugsweise von 45 bis 65°C zur Bildung von Neopentylglykol und unter Nutzung des bereits anwesenden Formaldehyds und des gegebenenfalls zugesetzten Formaldehyds betrieben werden. Das alkalische Reaktionsgemisch fließt aus der zweiten Reaktionszone über Leitung (10) in einen Behälter (11), in dem es durch Säurezugabe auf einen pH-Wert von 4 bis 7 eingestellt wird. Die Säurezugabe, beispielsweise die Zugabe von Ameisensäure oder Essigsäure, erfolgt über Leitung (12). Anschließend wird das mit Säure behandelte Gemisch über Leitung (13) auf eine Destillationseinrichtung (14) zur Abtrennung des überschüssigen Formaldehyds in Form einer verdünnten wässrigen Lösung gegeben. Bei der Destillationseinrichtung (14) kann es sich beispielsweise um eine konventionelle Destillationskolonne handeln. Mögliche Sumpftemperaturen liegen von 80 bis 160°C und mögliche Anlagendrücke von 300 hPa bis 0,3 MPa. Die wässrige Formaldehydlösung kann über Kopf (Leitung (15)) abgezogen werden. Am Sumpf fällt über Leitung (16) eine aufkonzentrierte wässrige Lösung mit dem Formiat, der anorganischen Base und ebenfalls den gewünschten Produkten Neopentylglykol und Trimethylolpropan an. Diese Lösung kann anschließend auf den oberen Teil der Extraktionskolonne (17) gegeben werden. Am Bodenbereich der Extraktionskolonne (17) kann das Extraktionsmittel über Leitung (18) herangeführt werden, das in Richtung des Kolonnenkopfes der aufgegebenen wässrigen produkthaltigen Lösung entgegenströmt. Am Boden der Extraktionskolonne (17) fließt die mit dem Formiat der anorganischen Base beladene wässrige Phase über die Leitung (19) ab. Zweckmäßigerweise wird oberhalb der Aufgabestelle der über Leitung (16) herangeführten produkthaltigen wässrigen Lösung noch zusätzlich Wasser über Leitung (20) auf die Extraktionskolonne aufgegeben, um die Formiatabtrennung zu vervollständigen. Das mit Produkt beladene Extraktionsmittel kann über Leitung (21) zur Entfernung des Extraktionsmittels auf eine Destillationseinrichtung (22), beispielsweise eine Abdampfkolonne, gegeben werden. Hier kann das Extraktionsmittel über Kopf abgezogen und über Leitung (23) mit frischem, über Leitung (18) herangeführtem Extraktionsmittel vereinigt und gemeinsam wieder in die Extraktionskolonne (17) zurückgefahren werden. Über den Sumpf der Destillationseinrichtung (22) wird das trimethylolpropan- und neopentylglykolhaltige Rohgemisch über Leitung (24) abgeführt und in einer dreistufigen Destillationsschaltung in die einzelnen Wertprodukte aufgetrennt.

### Apparativer Aufbau zur Trennung der Produkte

Zur Trennung der einzelnen Produkte wird das Rohprodukt auf den Mittelteil einer ersten Destillationseinrichtung (25) gegeben, die zur Leichtsiederabtrennung dient und aus der am Kopf über Leitung (26) Leichtsieder wie Reste an Extraktionsmittel, Wasser und leichtflüchtige Nebenprodukte, wie 2-Ethyl-1,3-propandiol, 2-Ethyl-2-Methyl-1,3-propandiol und das monozyklische Formal des Trimethylolpropans entfernt werden. Die erste Destillationseinrichtung (25) kann beispielsweise als Packungskolonne mit 10 bis 25 theoretischen Böden ausgestaltet werden und wird beispielsweise bei Sumpftemperaturen von 135 bis 150°C und bei Drücken von Normaldruck bis zu 60 hPa betrieben. Der Sumpfablauf aus der ersten Destillationseinrichtung wird anschließend über Leitung (27) auf den Mittelteil einer zweiten Destillationseinrichtung (28) geführt, die zur Hochsiederabtrennung dient. Die zweite Destillationseinrichtung kann beispielsweise als Packungskolonne ausgestaltet werden und bei Sumpftemperaturen von 200 bis 330°C und verminderten Drücken von 10 bis 90 hPa betrieben werden. Über Leitung (29) wird der hochsiederhaltige Sumpfstrom abgeführt, der beispielsweise thermisch verwertet werden kann. Am Kopf fällt ein Gemisch aus Neopentylglykol und Zwischensiedern an, die einen geringeren Siedepunkt als Trimethylolpropan aufweisen und über Leitung (30) abgeführt werden. Gereinigtes Trimethylolpropan wird der zweiten Destillationskolonne (28) über den Seitenstrom (31) entnommen und weist einen Gehalt von wenigstens 95 Gew.-% auf. Das über Leitung (30) aus der zweiten Destillationseinrichtung abgeführte Kopfprodukt wird anschließend auf den Mittelteil einer dritten Destillationseinrichtung (32) gegeben. Gereinigtes Neopentylglykol wird über die Leitung (33) abgeführt während die ebenfalls der dritten Destillationskolonne zugeführten Zwischensieder, die einen höheren Siedepunkt aufweisen als Neopentylglykol über Leitung (34) ausgeschleust werden. Die dritte Destillationseinrichtung kann beispielsweise als Fraktionierkolonne oder als Trennwandkolonne mit 40 bis 70 theoretischen Böden ausgestaltet werden und die bei Temperaturen von 210 bis 270°C und in einem Druckbereich von 600 hPa bis zu einem Überdruck von 0,2 MPa arbeitet. Der über Leitung (33) entnommene Stoffstrom weist einen Neopentylglykolgehalt von wenigstens 97 Gew.-% auf.

### Beispiele

Die Vorteile des erfindungsgemäßen Verfahrens werden anhand einer gekoppelten Umsetzung von n-Butanal (n) und iso-Butanal (i) zu jeweils Trimethylolpropan (TMP) und Neopentylglykol (NPG) gezeigt. Die Beispiele wurden unter anderem als Funktion der Zugabe, der Verfahrensführung (kontinuierlich vs. Batch) und des eingesetzten Reaktortyps aufgeschlüsselt:

| Beispiel | VerfahrensSchritt | Produkt | Edukt (Butanal) | Zugabe Edukte/Verfahren/Reaktor |
|---|---|---|---|---|
| 1-5 | 1 | TMP | n | Einzelsubstanz /Batch/ Kolben |
| 6-10 | 2 | NPG | i | Einzelsubstanz /Batch/ Kolben |
| 11 | 1 + 2 | TMP + NPG | n und i | Mischung /Batch/ Kolben |
| 12-14 | 1 + 2 | TMP + NPG | n und i | Getrennt/konti/Rührkesselkaskade |
| 15-18 | 1 + 2 | TMP + NPG | n und i | Getrennt /Batch/ Kolben |
| 19-22 | 1 + 2 | TMP + NPG | n und i | Getrennt /konti/ Rohr |

Die dargestellten Beispiele resultieren aus einer Versuchsmatrix, welche nach statistischen Methoden ausgewertet wurde. Hierdurch ergeben sich oftmals Beispiele in denen mindestens 2 oder mehr Parameter zu einem anderen Vergleichsbeispiel gleichzeitig geändert wurden.

### I. Beispiele 1-5 - Einzelherstellung TMP im Batch (Kolben)

Die Cannizzaro-Reaktion zur Herstellung von Trimethylolpropan wurde in einem 2 L - Vierhalskolben durchgeführt, der mit Rührer, Innenthermometer und zwei Tropfvorlagen ausgestattet wurde.

Entsprechend der in Tabelle 1 genannten Äquivalente wurde in einem Kolben eine wässrige Formaldehyd- (FA) Lösung vorgelegt sowie n-Butanal und KOH (45%ige Lösung) über einen Zeitraum von 10 Minuten gleichmäßig unter Rühren zugegeben. Mittels des Innenthermometers wird die Reaktionstemperatur überprüft und bei Bedarf mit einem Kältebad (Wasser/Eis-Mischung) auf der angegebenen Reaktionstemperatur gehalten. Nach der in Tabelle 1 angegebenen Reaktionszeit wird die Reaktion mittels Zugabe von Ameisen- oder Essigsäure beendet und auf einen pH-Wert von 6 eingestellt. Die jeweilige Reaktionsbedingungen und die Ergebnisse der Produktanalyse sind in der Tabelle 1 aufgeführt.

**Tabelle 1**

| **Beispiel** | | **1** | **1,1** | **2** | **3** | **3,1** | **4** | **5** |
|---|---|---|---|---|---|---|---|---|
| Reaktor | | Kolben | | | | | | |
| FA | (Gew-%) | 16 | | | 36 | | | 25 |
| FA | eq. | 6,6 | 6,6 | 3,2 | 6,6 | 6,6 | 6,6 | 6,6 |
| KOH | eq. | 1,1 | | | | 1,8 | 1,8 | 1,05 |
| n-Butanal | eq. | 1 | | | | | | |
| Reaktionszeit | [h] | 0,5 | 1 | 0,5 | 2 | 1 | | |
| T | [°C] | 50 | 60 | 50 | 60 | 50 | 60 | 50 |
| Abbruch über Zugabe von | | CHOOH | | | | | CH3COOH | CHOOH |

| Gaschromatographische Produktanalyse (Gew.-%) | | | | | | | | |
|---|---|---|---|---|---|---|---|---|
| Methanol | | 3,51 | 3,24 | 1,3 | 3,04 | 8,68 | 11,71 | 2,92 |
| i- + n-Butanal | | 0,10 | 0,02 | 0 | 0 | 0,07 | 0,03 | 0,02 |
| Nebenkomponenten | | 1,58 | 2,57 | 3,4 | 2,51 | 2,98 | 4,1 | 2,51 |
| TMP | | 90,37 | 88,28 | 83,3 | 84,89 | 80,47 | 76,63 | 86,3 |
| Formiate + Zwischenläufe | | 0,92 | 2,46 | 4,2 | 1,39 | 1,44 | 3,04 | 2,17 |
| Di-Trimethylolpropan | | 0,65 | 0,58 | 5,3 | 0,63 | 0,61 | 1,94 | 0,35 |
| Lineares bis-TMP-Formal | | 0,52 | 0,60 | 2,3 | 4,43 | 3,93 | 2,01 | 2,55 |
| Hochsieder | | 2,35 | 2,25 | 0,2 | 3,11 | 1,82 | 0,54 | 3,18 |
| | | | | | | | | |
| TMP-Selektivität (%) | | 93,3 | 91,1 | 84,4 | 87,3 | 87,8 | 86,6 | 88,7 |

Die Versuchsdaten der Tabelle 1 zeigen, dass die FA-Konzentration und die zugesetzten FA-Äquivalente einen entscheidenden Einfluss auf die Selektivität der Reaktion haben. Vergleicht man das Beispiel 1 mit dem Beispiel 2, welches mit einem kleineren Aldehyd-FA-Äquivalentverhältnis durchgeführt wurde, so ergibt sich ein deutlich geringerer Gesamtanteil an gewünschtem TMP bei Einsatz eines kleineren Aldehyd-FA-Äquivalentverhältnisses (Beispiel 2). Einen untergeordneten Einfluss der Reaktionstemperatur wird im Vergleich von Beispiel 1 mit 1,1 deutlich. Mit Zunahme der Reaktionstemperatur nimmt die Ausbeute an TMP ab. Ähnliche Ergebnisse werden auch bei dem Vergleich von Beispiel 3,1 mit 4 erhalten.

Der Einfluss der FA-Konzentration ergibt sich beispielsweise durch Vergleich von Beispiel 1 mit Beispiel 5. Hierbei spielt die Änderung der KOH-Äquivalente nur eine untergeordnete Rolle. Im Beispiel 5 wurde eine FA-Konzentration von 25 Gew.-% verwendet. Diese FA-Konzentration führt trotz einer doppelt so langen Reaktionsdauer zu einem TMP-Anteil von nur 86,3 Gew.-%. Eine Steigerung der FA-Konzentration auf 36 Gew.-% (Beispiel 3) führt zu einer weiteren Abnahme des TMP-Anteils. Ein zu hoher Anteil an Kaliumhydroxid wirkt sich negativ auf die ermittelte TMP-Ausbeute aus, wie man aus Beispiel 4 erkennen kann. Dies ergibt sich auch unter Berücksichtigung der Ergebnisse des Beispiels 3,1.

Für eine hohe Selektivität in der TMP-Herstellung sind eine niedrige Formalinkonzentration und ein hoher Formalinäquivalentenüberschuss günstig. Niedrigere Reaktionstemperaturen scheinen zudem zu höheren Selektivitäten zu führen. Im Falle des Einsatzes höherer Formalinkonzentrationen kann gleichzeitig der Einsatz höherer KOH-Äquivalente vorteilhaft sein. Insgesamt lassen sich durch diese Fahrweise Selektivitäten im Bereich von >89 Mol.-% erreichen.

### II. Beispiele 6 - 10 - Einzelherstellung NPG im Batch (Kolben)

Die Cannizzaro-Reaktion zur Herstellung von NPG wurde in einem 2 L - Vierhalskolben durchgeführt, der mit Rührer, Innenthermometer und zwei Tropfvorlagen ausgestattet wurde.

In dem Kolben wurden entsprechend der in Tabelle 2 genannten Äquivalente an FA vorgelegt und über einen Zeitraum von 10 Minuten das iso-Butanal und das Kaliumhydroxid (45%ige Lösung) entsprechend der in Tabelle 2 gegebenen Äquivalenten gleichmäßig unter Rühren zugegeben. Mittels des Innenthermometers wird die Reaktionstemperatur überprüft und bei Bedarf mit einem Kältebad (Wasser/Eis-Mischung) auf der angegebenen Reaktionstemperatur gehalten. Nach der in Tabelle 2 angegebenen Reaktionszeit wird die Reaktion mittels Zugabe von Ameisensäure oder Essigsäure beendet und auf einen pH-Wert von 6 eingestellt. Die jeweiligen Reaktionsbedingungen und die Ergebnisse der Produktanalyse sind in der Tabelle 2 aufgeführt.

**Tabelle 2**

| **Beispiel** | | **6** | **7** | **8** | **9** | **10** |
|---|---|---|---|---|---|---|
| Reaktor | | Kolben | | | | |
| FA | (Gew-%) | 30 | 30 | 34 | 40 | 49 |
| FA | eq. | 2,5 | 5 | 3 | 2,5 | 3,5 |
| KOH | eq. | 1,1 | 1,1 | 1,2 | 1,1 | 1,45 |
| i-Butanal | eq. | 1 | | | | |
| Reaktionszeit | [h] | 6 | 6 | 4 | 1 | 0,25 |
| T | [°C] | 60 | 60 | 55 | 55 | 55 |
| Abbruch über Zugabe von | | CHOOH | CH3COOH | CHOOH | | |

| Gaschromatographische Produktanalyse (Gew.-%) | | | | | | |
|---|---|---|---|---|---|---|
| Methanol | | 5,76 | 7,48 | 6,1 | 3,24 | 4,73 |
| i- + n-Butanal | | 0,00 | 0,02 | 0,01 | 0,02 | 0 |
| Nebenkomponenten | | 0,72 | 1,5 | 0,21 | 0,18 | 0,56 |
| Hydroxypivaldehyde | | 0,00 | 2,34 | 0,01 | 0 | 0,01 |
| NPG | | 91,68 | 88,06 | 93,57 | 96,22 | 94,11 |
| Hochsieder | | 1,84 | 0,6 | 0,1 | 0,34 | 0,59 |
| | | | | | | |
| NPG-Selektivität (%) | | 97,5 | 98,4 | 99,7 | 99,5 | 99,0 |

Die Beispiele 6 - 10 belegen, dass die Durchführung der Cannizzaro-Reaktion in Gegenwart eines großen FA-Überschusses bei einem nur geringen Überschussanteil an Base zu einem geringeren Neopentylglykol-Anteil führt (Vergleich der Beispiele 6 und 7). Sehr lange Reaktionszeiten haben auch einen großen Einfluss auf den gefundenen Neopentylglykol Anteil, wie in den Beispielen 6 und 7 zu sehen ist, im Vergleich zu Beispiel 9 und 10. Allgemein können für eine hohe Selektivität vergleichsweise höhere Konzentrationen an FA-Lösung als bei der Herstellung von Trimethylolpropan eingesetzt werden.

Im Vergleich zur TMP-Synthese ist die erfindungsgemäße NPG-Herstellung auch unter Einsatz höherer Formalinkonzentrationen sehr selektiv. Man erhält mit 3 bis 3,5 Formalinäquivalenten sehr gute Selektivitäten von >98 Mol.-%. Ebenso kann es vorteilhaft sein, bei dem Einsatz höherer Formalinmengen auch die KOH-Äquivalentmenge zu erhöhen.

### III. Beispiel 11 -11,1 - NPG/TMP im Batch (Kolben) - Simultanfahrweise

Die Cannizzaro-Reaktion zur simultanen Herstellung von Neopentylglykol und Trimethylolpropan wurde in einem 2 L - Vierhalskolben durchgeführt, der mit Rührer, Innenthermometer und zwei Tropfvorlagen ausgestattet wurde.

In dem Kolben wurden entsprechend der in Tabelle 3 genannten Äquivalente an FA vorgelegt und über einen Zeitraum von 10 Minuten eine Mischung der beiden Reaktanden n- und iso-Butanal über einen Tropftrichter sowie Kaliumhydroxid (45%ige Lösung) entsprechend der in Tabelle 3 gegebenen Äquivalente über einen separaten Tropftrichter gleichmäßig unter Rühren zugegeben. Mittels des Innenthermometers wird die Reaktionstemperatur überprüft und bei Bedarf mit einem Kältebad (Wasser/Eis-Mischung) auf der angegebenen Reaktionstemperatur gehalten. Nach der in Tabelle 3 angegebenen Reaktionszeit wird die Reaktion mittels Zugabe von Ameisensäure beendet und auf einen pH-Wert von 6 eingestellt. Die jeweiligen Reaktionsbedingungen und die Ergebnisse der Produktanalyse sind zusammen mit den Ergebnissen der Beispiele 12-14 in der Tabelle 3 aufgeführt und werden auch dort diskutiert.

### IV. Beispiel 12 -14 - NPG/TMP getrennte kontinuierliche Zugabe, Rührkesselkaskade

Die kontinuierliche und gleichzeitige Cannizzaro-Reaktion zur Herstellung von NPG und TMP wurde in einer Rührkesselkaskade mit zwei 1 L - Reaktoren durchgeführt, die jeweils mit Rührer, Innenthermometer, Überlauf, innenliegenden Strömungsbrechern, sowie Zuleitungen mit Förderpumpen für die Einsatzstoffe n-Butanal, iso-Butanal, Kaliumhydroxid-Lösung oder/und Formalin ausgestattet waren.

Im ersten Reaktor wurde über ein Tauchrohr die entsprechende Gesamtmenge an Formalin (6,6 Äquivalente bezogen auf n-Butanal) vorgelegt und über ein zweites Tauchrohr mit Kreuzstück die entsprechenden Äquivalente an n-Butanal und Kaliumhydroxid zugegeben.

Über die Höhe des Überlaufes im ersten Reaktor konnte die Füllhöhe und somit die entsprechende Verweilzeit, wie in Tabelle 3 angegeben, eingestellt werden.

Das am Überlauf des ersten Reaktors abgenommene Produkt wurde in den zweiten Reaktor geleitet, in dem je nach Vorgabe iso-Butanal und Kaliumhydroxid-Lösung entsprechend Tabelle 3 zugegeben wurde. Die Verweilzeit in diesem Reaktor wurde wiederum über ein Tauchrohr eingestellt.

**Tabelle 3**

| **Beispiel** | | **11** | **11,1** | **12** | **13** | **14** |
|---|---|---|---|---|---|---|
| Reaktor | | Kolben | | Rührkesselkaskade | | |
| FA | (Gew-%) | 25 | | | | |
| FA | eq. | 6,6 | | | | |
| KOH eq. | 1./2. Stufe | 2,2 | 2,2 | 2,2/0 | 1/1,2 | 0,7/1,5 |
| n-Butanal | eq. | 1 | | | | |
| i-Butanal | eq. | 1 | | | | |
| Reaktionszeit | [h] | 1* | 2* | 2* | | |
| T [°C] | 1./2. Stufe | 55 | 55/60 | 50/60 | 50/60 | 50/60 |
| Abbruch über Zugabe von | | CHOOH | | | | CH3COOH |

| Gaschromatographische Produktanalyse (Gew.-%) | | | | | | |
|---|---|---|---|---|---|---|
| Methanol | | 2,48 | 2,17 | 19,92 | 5,4 | 8,08 |
| i- + n-Butanal | | 0,16 | 0,08 | 0,13 | 0 | 0,27 |
| Nebenkomponenten | | 0,20 | 1,13 | 2,97 | 0,64 | 0,54 |
| Hydroxypivaldehyde | | 0,00 | 0,00 | 0,94 | 0,67 | 0,58 |
| NPG | | 44,79 | 42,72 | 13,38 | 42,65 | 34,25 |
| TMP | | 45,56 | 47,05 | 24,47 | 41,57 | 42,93 |
| Formiate/Zwischenläufe | | 3,56 | 3,48 | 36,94 | 4,97 | 9,63 |
| Di-TMP | | 1,12 | 1,03 | 0,36 | 1,01 | 1,17 |
| lineares bis-TMP-Formal | | 1,74 | 1,61 | 0,59 | 2,69 | 1,91 |
| Hochsieder | | 0,39 | 0,73 | 0,3 | 0,4 | 0,64 |
| | | | | | | |
| TMP-Selektivität (%) | | 87,9 | 89,4 | 53,8 | 88,4 | 84,2 |
| NPG-Selektivität (%) | | 99,3 | 97,0 | 52,7 | 91,3 | 86,9 |

| | | | | | | |
|---|---|---|---|---|---|---|
| * = Gesamtreaktionszeit als Summe der TMP- und NPG-Stufe | | | | | | |

Es wurde gefunden, dass in einer Rührkesselkaskade prinzipiell die beiden gewünschten Produkte unter Ausnutzung eines FA-Überschusses hergestellt werden können, jedoch wurde auch ein erhöhter Anfall an Formiaten und Zwischenläufen im Vergleich zur gleichzeitigen Herstellung in einem Eintopfverfahren verzeichnet (Beispiel 11-11,1). Dies ist an den angegebenen Selektivtäten zu ersehen, welche bereits im angestrebten Bereich für TMP (∼89%) und NPG (∼98%) liegen.

### V. Beispiel 15 -18 - NPG/TMP getrennte Zugabe Batch, Kolben

Die Cannizzaro-Reaktion zur Herstellung von Neopentylglykol und Trimethylolpropan wurde in einem 2 L - Vierhalskolben durchgeführt, der mit Rührer, Innenthermometer und zwei Tropfvorlagen ausgestattet wurde.

Zuerst wurde, analog den Beispielen 1 bis 5, Trimethylolpropan hergestellt. Hierzu wurde die entsprechende Menge an Formalin (6,6 Äquivalente bezogen auf n-Butanal) entsprechend Tabelle 4 vorgelegt und über einen Zeitraum von 10 Minuten n-Butanal und Base (Kaliumhydroxid/NaOH, >40%ige Lösung) entsprechend der in Tabelle 4 angegebenen Äquivalenten gleichmäßig unter Rühren zugegeben. Mittels des Innenthermometers wird die Reaktionstemperatur überprüft und bei Bedarf mit einem Kältebad (Wasser/Eis-Mischung) auf der angegebenen Reaktionstemperatur gehalten. In der 2. Stufe wird nach der in Tabelle 4 angegebenen halben Reaktionszeit eine entsprechende Menge an iso-Butanal und gegebenenfalls Kaliumhydroxid/Natriumhydroxid-Lösung innerhalb von 10 Minuten zugegeben und für eine weitere angegebene halbe Reaktionszeit gerührt. Die Reaktion wird mittels Zugabe von Ameisensäure oder Essigsäure beendet und auf einen pH-Wert von 6 eingestellt. Die jeweiligen Reaktionsbedingungen und die Ergebnisse der Produktanalyse sind in der Tabelle 4 aufgeführt.

**Tabelle 4**

| **Beispiel** | | **11,1** | **15** | **16** | **17** | **18** |
|---|---|---|---|---|---|---|
| Reaktor | | Kolben | | | | |
| FA | (Gew-%) | 25 | 30 | 25 | | |
| FA | eq. | 6,6 | | | | |
| Base | | KOH | | | | NaOH |
| Basen eq. | 1./2. Stufe | 2,2 | 2,2/0 | 1,1/1,1 | 1,1/1,1 | 1,1/1,1 |
| n-Butanal | eq. | 1 | | | | |
| i-Butanal | eq. | 1 | | | | |
| Reaktionszeit | [h] | 2* | | | | |
| T [°C] | 1./2. Stufe | 55/60 | 40/50 | 60/60 | 55/60 | 55/60 |
| Abbruch über Zugabe von | | CHOOH | | | | CH3COOH |

| Gaschromatographische Produktanalyse (Gew.-%) | | | | | | |
|---|---|---|---|---|---|---|
| Methanol | | 2,17 | 9,36 | 3,47 | 2,93 | 2,96 |
| i- + n-Butanal | | 0,08 | 6,57 | 0,3 | 0,11 | 0 |
| Nebenkomponenten | | 1,13 | 0,18 | 0,42 | 0,27 | 0,56 |
| Hydroxypivaldehyde | | 0,00 | 13,98 | 0 | 0 | 0 |
| NPG | | 42,72 | 12,16 | 49,05 | 49,46 | 45,64 |
| TMP | | 47,05 | 51,28 | 43,21 | 44,8 | 45,71 |
| Formiate/Zwischenläufe | | 3,48 | 4,17 | 1,4 | 0,76 | 1,22 |
| Di-TMP | | 1,03 | 0,33 | 0,3 | 0,22 | 0,86 |
| Lineares bis-TMP-Formal | | 1,61 | 0,66 | 1,24 | 0,58 | 2 |
| Hochsieder | | 0,73 | 1,31 | 0,61 | 0,87 | 1,05 |
| | | | | | | |
| TMP-Selektivität (%) | | 89,42 | 95,07 | 94,56 | 96,33 | 92,15 |
| NPG-Selektivität (%) | | 97,01 | 80,16 | 97,13 | 98,22 | 96,55 |

| | | | | | | |
|---|---|---|---|---|---|---|
| * = Gesamtreaktionszeit als Summe der TMP- und NPG-Stufe | | | | | | |

Eine Reaktionslösung mit einem hohen Produktanteil an Neopentylglykol und Trimethylolpropan wird beispielsweise durch die Reaktionsbedingungen des Beispiels 17 erhalten. Insbesondere ist hervorzuheben, dass der Anteil an Formiaten/Zwischenläufen in diesem Beispiel auf einen Wert von unter 1 Gew.-% abgesenkt werden konnte. Zudem stellte es sich als vorteilhaft heraus, dass die notwendige Basenmenge jeweils in der entsprechenden Reaktionsstufe zugefügt wird (Vergleich Beispiel 15 mit Beispiel 16-18).

Vergleicht man Beispiel 11,1 mit den Beispielen 15-18, ergibt sich für die Beispiele 15-18 eine wesentlich höhere Selektivität für TMP. Unter Berücksichtigung der Versuche 1-5 (nur TMP-Synthese) ergibt sich für die Beispiele mit einer konsekutiven TMP/NPG Fahrweise, unerwarteter Weise ein Selektivitätsvorteil. Dieser kann durch eine verminderte Bildung an linearen TMP-Formalen verursacht sein und ist ein klares Indiz für die Vorteilhaftigkeit einer konsekutiven Fahrweise. In den optimierten Versuchseinstellungen der Beispiele 17 und 18 können gleich gute NPG-Selektivitäten wie in Beispiel 11,1 erreicht werden.

### VI. Beispiel 19 - 22 - NPG/TMP getrennte kontinuierliche Zugabe, Rohrreaktor

Die Cannizzaro-Reaktion zur gekoppelten Herstellung von Neopentylglykol und Trimethylolpropan wurde in einem Rohrreaktor mit einer Länge von 175 m und einem Rohrinnendurchmesser von 3 mm durchgeführt. Das gesamte Rohr ist in zwei Rohrreaktorabschnitte unterteilt, welche jeweils mit Hilfe eines Heizbades auf die gewünschte Reaktionstemperatur eingestellt wurde.

Die Zugabe der Edukte erfolgte über Kreuzstücke am Rohrreaktoreingang. Die Gesamtmenge an FA wurde am Anfang des Rohrreaktors eingespeist. Zur Erhöhung der Ausbeute an Trimethylolpropan erfolgte die Zugabe an n-Butanal und Kaliumhydroxid /Natriumhydroxid in der Trimethylolpropan-Reaktionsstufe (1. Reaktorabschnitt) wahlweise stufenweise (Vergleich Beispiele 19 mit 22). Vor dem zweiten Reaktorrohrabschnitt wurde dann iso-Butanal und Kaliumhydroxid /Natriumhydroxid-Lösung zudosiert, so dass Neopentylglykol durch Verbrauch des überschüssigen Formalins im zweiten Rohrreaktorabschnitt entsteht. Es werden Proben aus den Auffangbehältern gezogen und die Reaktion wird mit Ameisensäure gequenscht. Die jeweilige Reaktionsbedingungen und die Ergebnisse der Produktanalyse sind in der Tabelle 5 aufgeführt.

**Tabelle 5**

| **Beispiel** | | **19** | **20** | **21** | **22** |
|---|---|---|---|---|---|
| Reaktor | | Rohr | | | |
| FA | (Gew.-%) | 25 | | | |
| FA | eq. | 6,6 | | | |
| Base | | KOH | | | |
| Basen eq. | 1./2. Stufe | 1,1/1,1 | | | |
| n-Butanal | eq. | 1 | | | 1 (sukz.) |
| i-Butanal | eq. | 1 | | | |
| Verweilzeit | [min] | 40 | | | |
| T [°C] | 1./2. Stufe | 40/60 | 50/50 | 20/30 | 40/60 |
| Abbruch über Zugabe von | | CHOOH | | | |

| Gaschromatographische Produktanalyse (Gew.-%) | | | | | |
|---|---|---|---|---|---|
| Methanol | | 4,39 | 3,30 | 2,18 | 3,10 |
| i- + n-Butanal | | 0,17 | 0,14 | 0,15 | 0,03 |
| Nebenkomponenten | | 2,17 | 0,73 | 2,14 | 0,70 |
| Hydroxypivaldehyde | | 0 | 0 | 0 | 0 |
| NPG | | 42,39 | 48,29 | 44,21 | 44,33 |
| TMP | | 49,28 | 46,43 | 50,27 | 51,01 |
| Formiate/Zwischenläufe | | 2,17 | 0,61 | 2,14 | 0,70 |
| Di-TMP | | 0,20 | 0,01 | 0,00 | 0,09 |
| n bis-TMP-Formal | | 0,04 | 0,35 | 0,00 | 0,01 |
| Hochsieder | | 0,49 | 0,50 | 0,09 | 0,20 |
| | | | | | |
| TMP-Selektivität (%) | | 92,87 | 96,34 | 93,92 | 97,59 |
| NPG-Selektivität (%) | | 95,28 | 98,13 | 95,89 | 98,47 |

| | | | | | |
|---|---|---|---|---|---|
| * = Gesamtreaktionszeit als Summe der TMP- und NPG-Stufe; (sukz.) = sukzessive Zugabe an n-Butanal | | | | | |

Der Vergleich der Produktanalyse für TMP und NPG (Versuche 19-22) zeigt, dass vergleichbar gute Ergebnisse wie im Kolbenreaktor erzielt werden können (Versuche 15-18). Die Umsetzung eines diskontinuierlichen Kolbenversuchs in die kontinuierliche Fahrweise entspricht der Durchführung im Rohrreaktor mit stufenweiser Zugabe. Zudem kann im ersten Verfahrensschritt bei einfacher Zugabe eine Selektivität von 92,9-96,3% für TMP und 95,3-98,1% für NPG erreicht werden (Versuche 19-21). Im Vergleich dazu zeigt Beispiel 22 den positiven Effekt einer stufenweisen Zugabe des n-Butanals. Durch die stufenweise Zugabe von n-Butanal in der ersten Reaktionsstufe bei verminderter Reaktionstemperatur (40 °C) (Versuch 22) können für beide Produkte TMP (97,6%) und NPG (98,5%) hervorragende Selektivitäten erreicht werden. Der zweite Verfahrensschritt kann wiederum bei höheren Temperaturen (60 °C) durchgeführt werden, ohne dass man hierbei eine Verringerung der Selektivität für beide Produkte feststellt. Die erhöhte Temperatur ist förderlich für die Realisierung eines Vollumsatzes bei vermindertem Reaktionsvolumen. In allen bisherigen Beispielen konnte in Kombination solch eine hohe Gesamtselektivität und einhergehend Gesamtausbeute an TMP und NPG nicht gezeigt werden.

### VII. Betrachtung der Energieeinsparung

Die Verwertung des nicht umgesetzten Formaldehyds durch den 2. Verfahrensschritt führt unmittelbar dazu, dass der Strom des überschüssigen Formaldehyds, der im Anschluss an die Reaktion abgetrennt wird, kleiner ausfällt. Dies führt unmittelbar zu einer Energieeinsparung, wobei die Energieeinsparung und die abzutrennende Formaldehydmenge nahezu proportional sind, da Formalin zu einer leichten Siedepunkterniedrigung führt.

An einer Beispielrechnung für die Produktion von 1 kmol Trimethylolpropan und 1 kmol Neopentylglykol soll die Energieeinsparung aufgezeigt werden. Bei der Verwendung einer Formalinlösung mit 20 Gew.-% für Trimethylolpropan und 35 Gew.-% für Neopentylglykol mit den jeweiligen Äquivalenten wie in Tabelle 6 angegeben, beträgt der Massenstrom an Wasser und Formalin 1257 kg, der nach Reaktionsende im Falle einer chargenweisen Fahrweise abgetrennt werden muss, während bei einer konsekutiven simultanen Fahrweise mit einer FA-Lösung von 20 Gew.-% nur 957 kg anfallen. Bei Verwendung von 30 bar Heizdampf und unter Beachtung der darin enthaltenen Energie, ergibt sich letztlich eine Energieeinsparung von 24%. Bei der konsekutiv simultanen Fahrweise verbleiben am Ende noch 1,2 eq. nicht abreagiertes Formaldehyd (3,8 Massen-%), während bei einer getrennten, chargenweisen Herstellung 4,7 eq. Formaldehyd (3,4 eq. bei TMP und 1,3 eq. bei NPG) verbleiben (11,9 Massen-%).

**Tabelle 6**

| Fahrweise | Getrennte Herstellung | Gekoppelte Herstellung |
|---|---|---|
| | TMP: 20 Gew.-% Formalin, 6,6 eq. | 20 Gew.-% Formalin, 6,6 eq. |
| | NPG: 35 Gew.-% Formalin, 3,5 eq. | |
| Massenstrom H2O+FA/kg | 1257 | 957 |
| Benötigte Energie/ kJ (Massenstrom x spez. Verdampfungsenthalpie) | 2,54·10⁶ | 2,04·10⁶ |
| Unterschied in % | 24 | |

## Patentansprüche

1. Verfahren zur simultan konsekutiven Herstellung mindestens zweier unterschiedlicher Polyole durch Umsetzung aliphatischer Aldehyde mit Formaldehyd in Gegenwart einer anorganischen Base,
**dadurch gekennzeichnet, dass** in einem
1. Verfahrensschritt ein aliphatischer C2-C9 Aldehyd mit Formaldehyd in Gegenwart einer anorganischen Base zur Reaktion gebracht und ohne Aufarbeitung der Reaktionslösung in einem
2. Verfahrensschritt die Reaktionslösung aus dem 1. Verfahrensschritt unter Zugabe zumindest eines vom Aldehyd des 1. Verfahrensschrittes unterschiedlichen aliphatischen C2-C9 Aldehyds weiter zur Reaktion gebracht wird.

2. Verfahren nach Anspruch 1, wobei im 1. Verfahrensschritt ein an 2-Position nicht-verzweigter aliphatischer C2-C9 Aldehyd und im 2. Verfahrensschritt ein in 2-Position verzweigter aliphatischer C2-C9 Aldehyd zur Reaktion gebracht werden.

3. Verfahren nach einem der Ansprüche 1 oder 2, wobei der im 1. Verfahrensschritt eingesetzte Aldehyd aus der Gruppe bestehend aus n-Propanal, n-Butyraldehyd, Valeraldehyd ausgesucht und der im 2. Verfahrensschritt eingesetzte Aldehyd iso-Butyraldehyd ist.

4. Verfahren nach einem der vorhergehenden Ansprüche, wobei der 1. und der 2. Verfahrensschritt bei einer Temperatur von größer oder gleich 10 °C und kleiner oder gleich 105 °C durchgeführt wird.

5. Verfahren nach einem der vorhergehenden Ansprüche, wobei im 2. Verfahrensschritt zusätzlich eine anorganische Base zur Reaktionslösung zugegeben wird.

6. Verfahren nach Anspruch 5, wobei im 1. und 2. Verfahrensschritt dieselbe anorganische Base eingesetzt wird.

7. Verfahren nach einem der vorhergehenden Ansprüche, wobei die anorganische Base ausgesucht ist aus der Gruppe bestehend aus NaOH, KOH, Ca(OH)2 oder Mischungen daraus.

8. Verfahren nach einem der vorhergehenden Ansprüche, wobei das Molverhältnis anorganischer Base zu aliphatischem Aldehyd in den beiden Verfahrensschritten größer oder gleich 1 zu 1 und kleiner oder gleich 1,6 zu 1 beträgt.

9. Verfahren nach einem der vorhergehenden Ansprüche, wobei der Formaldehyd im 1. Verfahrensschritt in Form einer wässrigen Formaldehydlösung mit einem Formaldehydgehalt von größer oder gleich 5 Gew.-% und kleiner oder gleich 50 Gew.-% zugegeben wird.

10. Verfahren nach einem der vorhergehenden Ansprüche, wobei das Molverhältnis Formaldehyd zu aliphatischem Aldehyd im 1. Verfahrensschritt größer oder gleich 3,1 zu 1 und kleiner oder gleich 12 zu 1 und das Molverhältnis Formaldehyd zu aliphatischem Aldehyd im 2. Verfahrensschritt größer oder gleich 2,1 zu 1 und kleiner oder gleich 7 zu 1 beträgt.

11. Verfahren nach einem der vorhergehenden Ansprüche, wobei Formaldehyd nur im 1. Verfahrensschritt zugegeben wird.

12. Verfahren nach einem der vorhergehenden Ansprüche, wobei die Zugabe mindestens eines Eduktes im 1. und/oder im 2. Verfahrensschritt stufenweise erfolgt.

13. Verfahren nach Anspruch 12, wobei der aliphatische Aldehyd im 1. Verfahrensschritt stufenweise der Reaktionsmischung zugegeben wird.

14. Verfahren nach einem der vorhergehenden Ansprüche, wobei der Umsatz an Aldehyd in der Reaktionsmischung am Ende des 1. Verfahrensschrittes größer oder gleich 50% beträgt.

15. Verfahren nach einem der vorhergehenden Ansprüche, wobei das durchgesetzte Eduktvolumen pro Reaktorvolumen und Zeit (V/Vh) in beiden Verfahrensschritten größer oder gleich 0,3 und kleiner oder gleich 4,0 beträgt.

## Claims

1. Process for simultaneously consecutive preparation of at least two different polyols by reaction of aliphatic aldehydes with formaldehyde in the presence of an inorganic base,
**characterized in that**, in a
1st process step, an aliphatic C2-C9 aldehyde is reacted with formaldehyde in the presence of an inorganic base and, without workup of the reaction solution, in a 2nd process step, the reaction solution from the 1st process step is converted further with addition of at least one aliphatic C2-C9 aldehyde other than the aldehyde from the 1st process step.

2. Process according to Claim 1, wherein, in the 1st process step, an aliphatic C2-C9 aldehyde unbranched in the 2 position is reacted, and, in the 2nd process step, an aliphatic C2-C9 aldehyde branched in the 2 position.

3. Process according to either of Claims 1 or 2, wherein the aldehyde used in the 1st process step is selected from the group consisting of n-propanal, n-butanal, valeraldehyde, and the aldehyde used in the 2nd process step is i-butanal.

4. Process according to any of the preceding claims, wherein the 1st and 2nd process step are conducted at a temperature of greater than or equal 10°C and less than or equal 105°C.

5. Process according to any of the preceding claims, wherein, in the 2nd process step, an inorganic base is additionally added to the reaction solution.

6. Process according to claim 5, wherein the same inorganic base is used in the 1st and 2nd process step.

7. Process according to any of the preceding claims, wherein the inorganic base is selected from the group consisting of NaOH, KOH, Ca(OH)2 or mixtures thereof.

8. Process according to any of the preceding claims, wherein the molar ratio of inorganic base to aliphatic aldehyde in the two process steps is greater than or equal 1:1 and less than or equal 1.6:1.

9. Process according to any of the preceding claims, wherein the formaldehyde is added in the 1st process step in the form of an aqueous formaldehyde solution having a formaldehyde content of greater than or equal 5% by weight and less than or equal 50% by weight.

10. Process according to any of the preceding claims, wherein the molar ratio of formaldehyde to aliphatic aldehyde in the first process step is greater than or equal 3.1:1 and less than or equal 12:1 and the molar ratio of formaldehyde to aliphatic aldehyde in the 2nd process step is greater than or equal 2.1:1 and less than or equal 7:1.

11. Process according to any of the preceding claims, wherein formaldehyde is added only in the 1st process step.

12. Process according to any of the preceding claims, wherein the addition of at least one reactant in the 1st and/or in the 2nd process step is effected stepwise.

13. Process according to Claim 12, wherein the aliphatic aldehyde in the 1st process step is added stepwise to the reaction mixture.

14. Process according to any of the preceding claims, wherein the conversion of aldehyde in the reaction mixture at the end of the 1st process step is greater than or equal 50%.

15. Process according to any of the preceding claims, wherein the reactant volume throughput per unit reactor volume and time (V/Vh) in the two process steps is greater than or equal 0.3 and less than or equal 4.0.

## Revendications

1. Procédé de production simultanée consécutive d'au moins deux polyols différents par réaction d'aldéhydes aliphatiques avec du formaldéhyde en présence d'une base inorganique,
**caractérisé en ce que** dans une
1^{ère} étape de procédé, un aldéhyde aliphatique en C2 à C9 est mis en réaction avec du formaldéhyde en présence d'une base inorganique et sans traitement de la solution réactionnelle et, dans une
2^{ème} étape de procédé, la solution réactionnelle provenant de la 1^{ère} étape de procédé est mise en réaction une nouvelle fois, moyennant l'ajout d'au moins un aldéhyde aliphatique en C2 àC9 différent de l'aldéhyde de la 1^{ère} étape de procédé.

2. Procédé selon la revendication 1, dans lequel, dans la 1^{ère} étape de procédé, un aldéhyde aliphatique en C2 à C9 non ramifié en position 2 et dans la 2^{ème} étape, un aldéhyde aliphatique en C2 à C9 ramifié en position 2 sont mis en réaction.

3. Procédé selon l'une des revendications 1 ou 2, dans lequel l'aldéhyde employé dans la 1^{ère} étape de procédé est choisi dans le groupe constitué du n-propanal, du n-butyraldéhyde, du valéraldéhyde, et l'aldéhyde employé dans la 2^{ème} étape de procédé est l'iso-butyraldéhyde.

4. Procédé selon l'une des revendications précédentes, dans lequel, la 1^{ère} étape et la 2^{ème} étape de procédé sont effectuées à une température supérieure ou égale à 10 °C et inférieure ou égale à 105 °C.

5. Procédé selon l'une des revendications précédentes, dans lequel une base inorganique est en outre ajoutée à la solution réactionnelle dans la 2^{ème} étape de procédé.

6. Procédé selon la revendication 5, dans lequel la même base inorganique est employée dans les 1^{ère} et 2^{ème} étapes de procédé.

7. Procédé selon l'une des revendications précédentes, dans lequel la base inorganique est choisie dans le groupe constitué de NaOH, de KOH, de Ca(OH)₂ ou de mélanges de celles-ci.

8. Procédé selon l'une des revendications précédentes, dans lequel le rapport molaire de la base inorganique sur l'aldéhyde aliphatique est supérieur ou égal à 1 sur 1 ou est inférieur ou égal à 1,6 sur 1 dans les deux étapes de procédé.

9. Procédé selon l'une des revendications précédentes, dans lequel le formaldéhyde dans la 1^{ère} étape de procédé est ajouté sous forme d'une solution de formaldéhyde aqueuse avec une teneur en formaldéhyde supérieure ou égale à 5 % en poids ou inférieure ou égale à 50 % en poids.

10. Procédé selon l'une des revendications précédentes, dans lequel le rapport molaire du formaldéhyde sur l'aldéhyde aliphatique dans la 1^{ère} étape de procédé est supérieur ou égal à 3,1 sur 1 et inférieur ou égal à 12 sur 1 et le rapport molaire du formaldéhyde sur l'aldéhyde aliphatique dans la 2^{ème} étape de procédé est supérieur ou égal à 2,1 sur 1 et inférieur ou égal à 7 sur 1.

11. Procédé selon l'une des revendications précédentes, dans lequel on ajoute du formaldéhyde uniquement dans la 1^{ère} étape de procédé.

12. Procédé selon l'une des revendications précédentes, dans lequel l'ajout d'au moins un produit de départ dans la 1^{ère} étape et/ou dans la 2^{ème} étape de procédé a lieu de manière échelonnée.

13. Procédé selon la revendication 12, dans lequel l'aldéhyde aliphatique est ajouté de manière échelonnée au mélange réactionnel dans la 1^{ère} étape de procédé.

14. Procédé selon l'une des revendications précédentes, dans lequel le degré de conversion en aldéhyde dans le mélange réactionnel à la fin de la 1^{ère} étape de procédé est supérieur ou égal à 50 %.

15. Procédé selon l'une des revendications précédentes, dans lequel le volume de produit de départ converti par volume de réacteur et en temps (V/Vh) dans les deux étapes de procédé est supérieur ou égal à 0,3 et inférieur ou égal à 4,0.
